(19) Europäisches Patentamt
European Patent Office
Office européen des brevets

(11) **EP 3 904 863 A1**

(12) **EUROPEAN PATENT APPLICATION**
published in accordance with Art. 153(4) EPC

(43) Date of publication:
**03.11.2021 Bulletin 2021/44**

(51) Int Cl.:
*G01N 21/49* (2006.01)    *G16H 20/60* (2018.01)

(21) Application number: **19901820.1**

(86) International application number:
**PCT/JP2019/050982**

(22) Date of filing: **25.12.2019**

(87) International publication number:
**WO 2020/138225 (02.07.2020 Gazette 2020/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2018 JP 2018243387**

(71) Applicant: **Medical Photonics Co., Ltd.
Sapporo-shi, Hokkaido 001-0021 (JP)**

(72) Inventor: **IINAGA, Kazuya
Sapporo-shi, Hokkaido 001-0021 (JP)**

(74) Representative: **Vossius & Partner
Patentanwälte Rechtsanwälte mbB
Siebertstraße 3
81675 München (DE)**

(54) **ABSORBED CALORIE MEASURING DEVICE, ABSORBED CALORIE MEASURING METHOD, AND ABSORBED CALORIE MEASURING PROGRAM**

(57)    [Problem] To provide a device with which it is possible to learn calorie consumption
state by performing monitoring.

[Solution] This absorbed calorie measuring device comprises: an irradiating unit which irradiates a subject with light; a light receiving
unit which detects the intensity of reception light that has passed through the subject
and emitted outside the subject; and a control unit which calculates absorbed calorie
from the reception light intensity.

FIG. 1

EP 3 904 863 A1

**Description**

Technical Field

The present invention relates to an absorbed calorie measuring device, an absorbed calorie measuring method, and an absorbed calorie measuring program.

Background Art

Recent years have seen an increasing demand for obtaining meal calorie information in a simple method for the purpose of health management. For example, Patent Literature 1 indicated below discloses an apparatus for obtaining calorie information from the reflected light of food.

Citation List

Patent Literature

   Patent Literature 1: JP 2016-81407 A
   Patent Literature 2: JP 2012-203834 A

Summary of the Invention

Technical Problem

However, even though Patent Literature 1 can obtain the calories of food, not all of the ingested calories is actually absorbed. The calories are partially absorbed and there are also individual differences in the absorption rate.
Further, Patent Literature 2 discloses a menu proposal program for controlling ingested calories. However, these inventions analyze an image of food, thereby presenting a user with the calories of food and drinks.
There are also applications that calculate consumed calories from the amount of exercise, but it is difficult to immediately monitor the metabolic effects of exercise, and it is unknown whether or not the calories are actually consumed.
The present invention provides a device and method for measuring the amount of calories absorbed by a subject, and also provides a device and method for enabling the state of calorie consumption to be obtained by carrying out monitoring.

Solution to Problem

The present invention relates to an absorbed calorie measuring device which comprises an irradiation unit configured to irradiate a subject with light, a light receiving unit configured to detect an intensity of received light which passes through the subject and is emitted outside the subject, and a control unit configured to calculate absorbed calories from the intensity of received light.
Furthermore, the present invention relates to an absorbed calorie measuring program for causing a computer of an absorbed calorie measuring device to execute a process of calculating absorbed calories from an intensity of received light, the device comprising an irradiation unit configured to irradiate a subject with light and a light receiving unit configured to detect an intensity of received light which passes through the subject and is emitted outside the subject.
Furthermore, the present invention relates to an absorbed calorie measuring method for causing a computer of an absorbed calorie measuring device to execute a process of calculating absorbed calories from an intensity of received light, the device comprising an irradiation unit configured to irradiate a subject with light and a light receiving unit configured to detect an intensity of received light which passes through the subject and is emitted outside the subject.
Furthermore, the present invention relates to an absorbed calorie measuring device connected to a mobile terminal, comprising a control unit configured to calculate absorbed calories from the intensity of received light, the mobile terminal comprising an irradiation unit configured to irradiate a subject with light and a light receiving unit configured to detect an intensity of received light which passes through the subject and is emitted outside the subject.
Furthermore, the present invention relates to an absorption calorie measuring program for causing a computer of an absorbed calorie measuring device connected to a mobile terminal to execute a process of calculating absorbed calories from an intensity of received light, the mobile terminal comprising an irradiation unit configured to irradiate a subject with light and a light receiving unit configured to detect the intensity of received light which passes through the subject and is emitted outside the subject.

Furthermore, the present invention relates to an absorption calorie measuring method for causing a computer of an absorbed calorie measuring device connected to a mobile terminal to execute a process of calculating absorbed calories from an intensity of received light, the mobile terminal comprising an irradiation unit configured to irradiate a subject with light and a light receiving unit configured to detect the intensity of received light which passes through the subject and is emitted outside the subject.

Brief Description of Drawings

[Fig. 1] Fig. 1 is a schematic diagram of an absorbed calorie measuring device.
[Fig. 2] Fig. 2 is a diagram showing the position of a subject and an absorbed calorie measuring device at the time of measurement.
[Fig. 3] Fig. 3 is a block diagram of the absorbed calorie measuring device.
[Fig. 4] Fig. 4 is a diagram depicting the temporal changes of absorbed calories.
[Fig. 5] Fig. 5 is an operation screen displayed on an operation panel.
[Fig. 6] Fig. 6 is an operation screen displayed on the operation panel.
[Fig. 7] Fig. 7 is an operation screen displayed on the operation panel.
[Fig. 8] Fig. 8 is a flowchart of the operation of measuring absorbed calories.
[Fig. 9] Fig. 9 is a correlation diagram of the intensity of received light and TG.
[Fig. 10] Fig. 10 is a diagram showing the relationship between the intensity of received light and a distance from an irradiation position.
[Fig. 11] Fig. 11 is a correlation diagram of the intensity of received light and absorbed calories.
[Fig. 12] Fig. 12 is a schematic diagram of an absorbed calorie measuring system.
[Fig. 13] Fig. 13 is a block diagram of the absorbed calorie measuring device.
[Fig. 14] Fig. 14 is a flowchart of the operation of measuring absorbed calories.

Description of Embodiments

An embodiment will be described below with reference to the drawings. In the embodiment, an example in which a smartphone is used as the absorbed calorie measuring device will be described. However, the measuring device is not limited to the smartphone, and absorbed calories can be measured by a device comprising an irradiation unit and a light receiving unit.

An absorbed calorie measuring device 100 according to the embodiment comprises an irradiation unit 11, a light receiving unit 12, a storage unit 13, a control unit 14, and an operation panel unit 15.

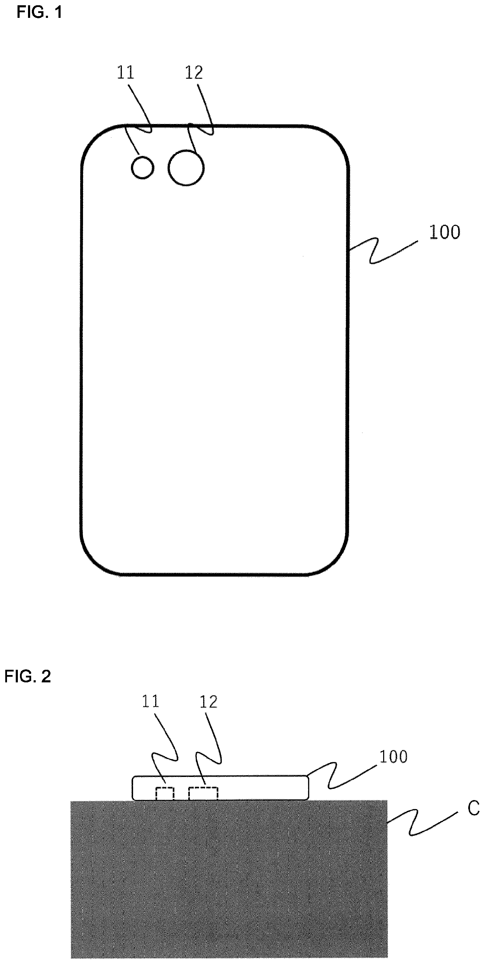

Fig. 1 is a diagram schematically showing a configuration example of the absorbed calorie measuring device 100 according to the embodiment. As illustrated in Fig. 1, the absorbed calorie measuring device 100 is, for example, a mobile terminal such as a smartphone, a tablet, or a notebook computer. The absorbed calorie measuring device 100 has a network interface capable of performing wireless communication and loads a program stored in a storage medium to be loaded into a memory and causes a CPU (Central Processing Unit) to execute the program to make each function effective.

The irradiation unit 11 according to the embodiment is arranged at a predetermined distance from the light receiving unit 12. As shown in Fig. 2, the irradiation unit 11 irradiates a subject C with light. The irradiation unit 11 is, for example, a fluorescent lamp, an LED, a laser, an incandescent lamp, an HID, a halogen lamp, or the like. The illuminance of the irradiation unit 11 is controlled by the control unit 14. By increasing the irradiation intensity, the irradiation unit 11 can also be used to target veins and search for the positional information of the veins. In the embodiment, the irradiation unit 11 is a flash mounted on a smartphone, but is not limited to the flash, and it may include a device that can irradiate a subject with light.

As shown in Fig. 2, the light receiving unit 12 according to the embodiment receives the light which is irradiated by the irradiation unit 11 and is emitted from inside to outside the subject C. The light receiving unit 12 according to the embodiment is a CMOS. The light receiving unit 12 is not limited to the CMOS, and may be a CCD or a photodiode. The data of the intensity of received light obtained by the light receiving unit 12 is transmitted to the storage unit 13. A user uses the operation panel unit 15 to designate whether the obtained data of the intensity of received light is transmitted to the reference data area or the post-meal data area of the storage unit 13.

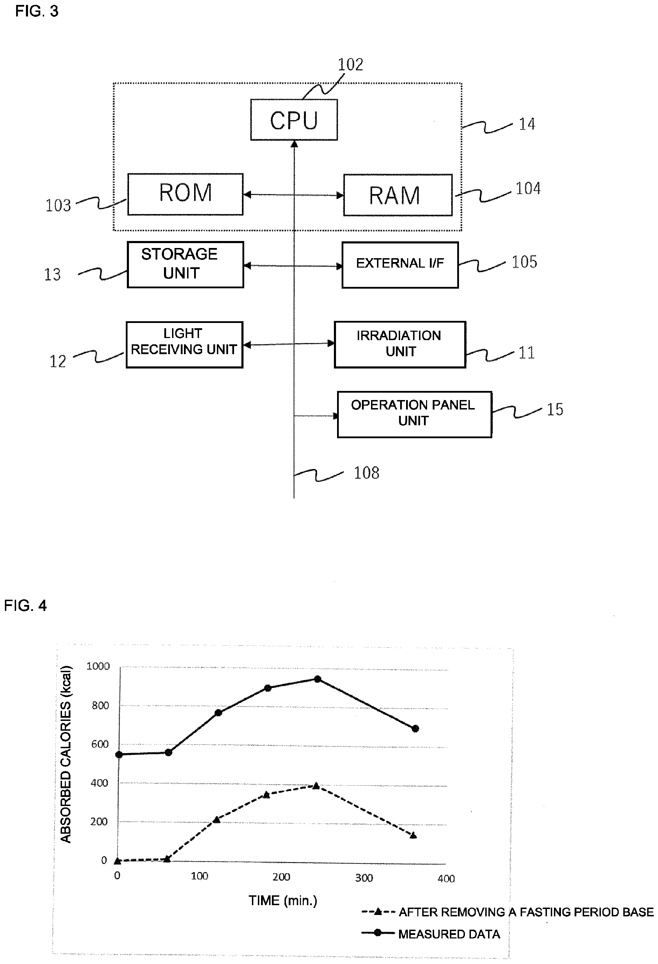

The configuration of a control system of the absorbed calorie measuring device 100 owned by the user is explained. Fig. 3 is a block diagram of the absorbed calorie measuring device 100 according to the embodiment. A CPU 102, a ROM (Read Only Memory) 103, a RAM (Random Access Memory) 104, an external I/F (Interface) 105, the irradiation unit 11, the light receiving unit 12, the storage unit 13, and the operation panel 15 are connected via a system bus 108. The CPU 102, the ROM 103, and the RAM 104 constitute the control unit 14.

The ROM 103 previously stores programs and thresholds to be executed by the CPU 102.

In the RAM 104, various memory areas, such as an area for developing a program to be executed by the CPU 102 and a work area for data processing by the program, are dynamically formed.

The storage unit 13 is a non-volatile storage device and is an internal storage such as an SSD (Solid State Drive) or an HDD (Hard Disc Drive).

The storage unit 13 has a reference data area configured to store calories absorbed in a fasting period which serve as a fasting period standard value. The storage unit 13 has a post-meal data area configured to store calories absorbed after a meal over the passage of time.

The storage unit 13 stores statistical data in which a correlation between absorbed calories and the intensity of received light is measured previously and converted into a calibration curve. In the embodiment, calories absorbed in a fasting period, calories absorbed after a meal, and statistical data are stored in the storage unit 13, but may be stored in the ROM 103 or the RAM 104.

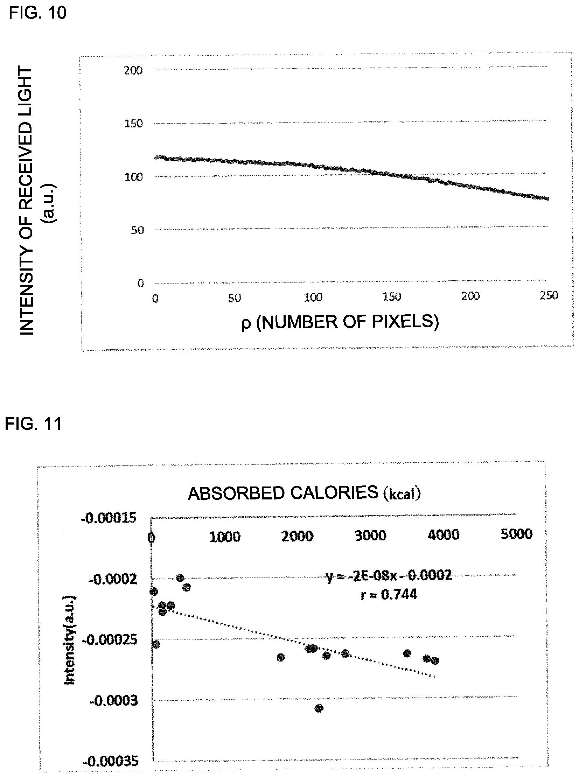

The control unit 14 calculates calories absorbed in a fasting period from an intensity of received light in a fasting period. The control unit 14 calculates calories absorbed after a meal from an intensity of received light after a meal. As shown in Fig. 11, there is a correlation between the intensity of received light and absorbed calories and therefore, the correlation between the intensity of received light and absorbed calories is measured in advance and statistical data (data based on, for example, the calibration curve y=-2E-08x-0.0002 in Fig. 11) is stored in the storage unit 13. Based on the statistical data stored in the storage unit 13, calories absorbed in a fasting period and calories absorbed after a meal are calculated from the intensity of received light.

The control unit 14 calculates net absorbed calories from the difference between the calories absorbed after a meal and the calories absorbed in a fasting period. As shown in Fig. 4, the temporal changes in the calories absorbed after a meal are represented by a solid graph line. However, if the calories absorbed in a fasting period are subtracted, the remaining calories are represented by a dotted graph line of Fig. 4, which represents the temporal changes in net absorbed calories. There are individual differences in the fasting period caloric values, but if the difference is taken, it becomes easier to make a comparison with the data of other people. In addition, it provides an effect that makes it easy to recognize the amount of absorption resulting from a meal. In the embodiment, the difference was taken, but a ratio may be taken.

In the embodiment, the calories absorbed in a fasting period and the calories absorbed after a meal are calculated individually, but obtaining calories is not limited to this calculation. The absorbed calories at 0 minutes of the calories absorbed after a meal in Fig. 4 may be deemed to be the calories absorbed in a fasting period (i.e., a standard value) and the above process may be performed.

How absorbed calories are measured when a commercially available smartphone is used as the absorbed calorie measuring device 100 according to the embodiment will be described. In the embodiment, changes in the turbidity of blood are converted into calories.

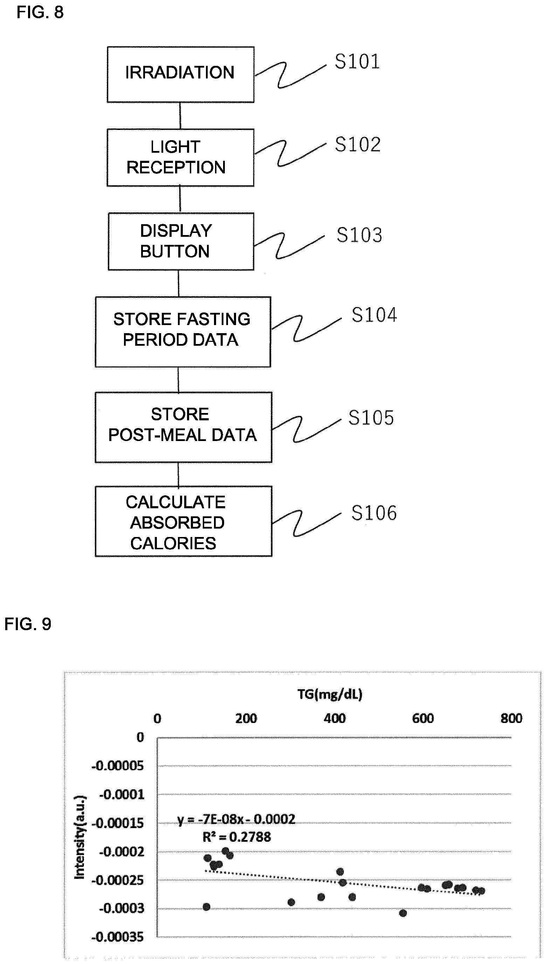

First, of the calories, particularly the calories of lipids were examined. It is known that when lipids are ingested, they are packaged into TG or chylomicron particles secreted to enter the blood circulation, and changes in the turbidity of blood then emerge as changes in the scattering coefficient of blood. From these changes in the scattering coefficient TG concentration can be obtained if a correlation coefficient of TG is 0.7 or greater in the case of use of near-infrared light. When the same experiment was conducted by using a commercially available smartphone, no correlation was obtained as shown in Fig. 9.

A close-up image was taken with a flash. The close-up image is shown in Fig. 10. The value of light proportional to a distance from the irradiation position is defined as intensity.

The reason for which no correlation was obtained is that the used light source emits visible light which exerts stronger effects on a living body in the same way as on both the scattering coefficient and the absorption coefficient than near-infrared light does. Furthermore, since the flash is basically intended to light up a subject even at night, its irradiation intensity is strong. In this case, it is desirable to set a longer distance between the incidence and the light receiving unit in order to satisfy the diffusion theory, but this distance is fixed on the device.

In addition, since the analysis for cameras such as smartphones is RGB analysis, poor correlation is considered to be attributable to an inability to perform detailed wavelength analysis.

Therefore, it is desirable to use a relatively simple fingertip in order to average as much biological noise as possible, but in the case of the forearm, it is also possible to average a position gap, etc. in a video mode.

Since the device configuration cannot be changed by commercially available smartphones, etc., it should be changed by software.

The present inventor used a commercially available smartphone and as a result of the examination of various indices such as calories, lipid concentration, particle size, and number of particles, he found that calories were most correlated to the intensity of received light.

Calculation of calories is based on 1g of lipid = 9kcal, and an increased TG concentration after a meal can be deemed to be the amount of absorbed lipids. In addition, in this embodiment, corrections depending on the volume of blood were made. The volume of blood is assumed to be 1/13 of the body weight. When the calories were multiplied by the volume of blood to obtain the absorbed calories of the entire body, the correlation coefficient was improved and as shown in Fig. 11, the correlation coefficient was 0.744 and good correlation was obtained. Fig. 11 is a diagram of correlation between the previously measured intensities of received light and absorbed calories. Fig. 11 shows a calibration curve obtained by linearly approximating the distribution of the obtained intensities of received light and absorbed calories. The approximation is not limited to linear approximation, but may also be curve approximation. By using this statistical data, absorbed calories can be calculated from the intensity of received light.

When ingested calories are known, this method enables an absorbed amount to be obtained. Therefore, it can also be used for a physical checkup and effective nutrition guidance.

The method of calculating absorbed calories is as follows. In the verification of the present invention, a subject was asked to ingest 160g of OFTT cream to conduct an experiment. The lipid component in 100g of OFTT cream is 32.9g. First, it is generally deemed that the calories of lipids are calculated based on "1g of lipid = 9kcal." Therefore, if the user ingests 160g of lipids alone, it can be understood that the load to the user was 473.8 kcal.

$$473.8 = 32.9g\ (W/V) \times 1.6\ (weight\ correction) \times 9\ (calorie\ conversion)$$

In addition, the ingested weight was calculated to be 52,640mg. The volume of blood is assumed to be 1/13 of the body weight. If a person weighs 60kg, his volume of blood is 4.6kg and his blood volume is 4.6L since in the case of blood, 1L=1kg. If a person weighs 100kg, his blood volume is about 7.7L as a result of the same calculation. When this is represented by the amount of TG change, the amount of TG increase in the case of 100% absorption can be calculated as follows:

60kg: about 1,144 mg/dL
100kg: about 684mg/dL

In addition, the absorbed calories are equal to $\Delta TG \times 9$.

The external I/F 105 is an interface for communicating with an external device. It is sufficient if the external I/F 105 is an interface that performs data communication with an external device. For example, the external I/F 105 may be a device (USB memory or the like) that is locally connected to an external device, or a network interface for communicating via a network. Furthermore, its data communication method may be Wi-Fi (registered trademark) communication or USB communication.

The fat absorbed by the body is packaged into particles called chylomicrons and enters the blood circulation, and changes in turbidity then are measured.

The operation panel 15 is a user interface in which a display unit and a touch panel are integrated. The operation panel 15 is arranged on the front of the main body of the absorbed calorie measuring device 100 and comprises the display unit including the touch panel. The control unit 14 controls the content displayed on the display unit of the operation panel 15. The operation panel 15 outputs information input through the touch panel to the control unit 14. The information of each process input from the operation panel 15 is stored in a predetermined area of the RAM 104 as process information.

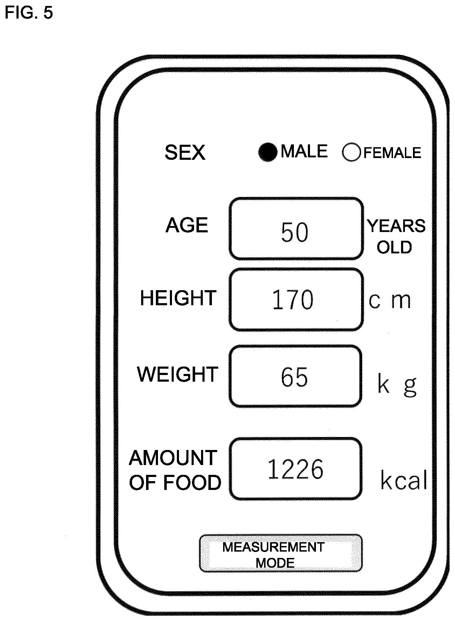

Fig. 5 is an input screen displayed on the operation panel 15. The user inputs the sex, age, height, weight, and amount of food in the operation panel 15. Measurement is started by pressing the measurement mode button.

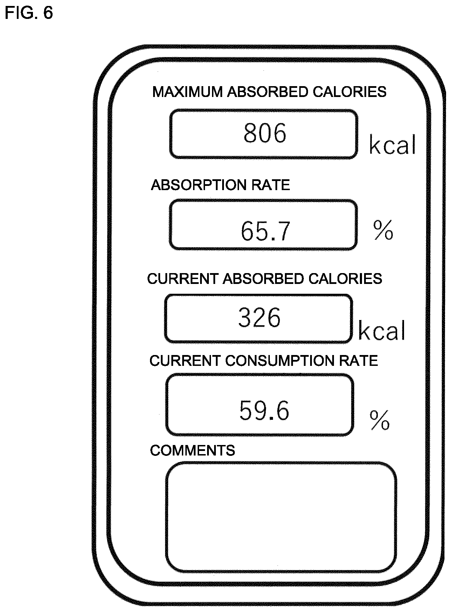

Fig. 6 is an output screen displayed on the operation panel 15. The operation panel 15 displays maximum absorbed calories, an absorption rate, current absorbed calories, a current consumption rate, and comments.

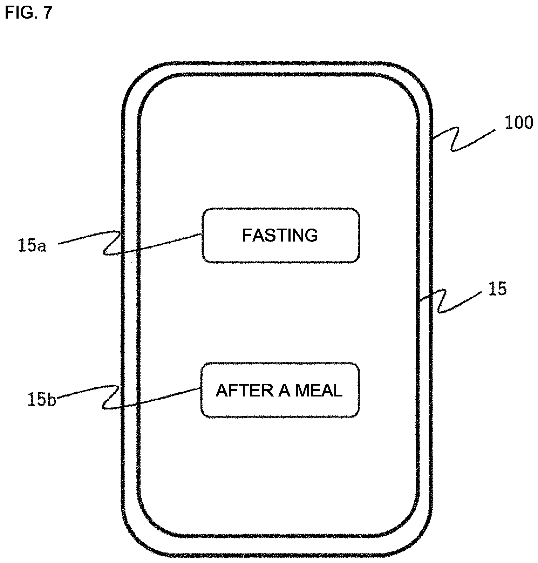

Fig. 7 shows an operation screen displayed on the operation panel 15. A fasting period button 15a is selected if the user is fasting. A post-meal button 15b is selected if the user has had a meal.

The absorbed calorie measuring device 100 which has the above configuration executes an absorbed calorie measuring process based on a preset program.

Fig. 8 is a flowchart showing the operation of the absorbed calorie measuring device 100 according to the embodiment. The irradiation unit 11 irradiates a subject with irradiation light (STEP 101). The light receiving unit 12 receives light emitted from inside to outside the subject (STEP 102). The control unit 14 displays the fasting period button 15a and the post-meal button 15b on the operation panel 15 (STEP 103). If the fasting period button 15a is selected, the control unit 14 calculates calories absorbed in a fasting period from an intensity of light received by the light receiving unit 14 and stores the calorie information in the reference data area of the storage unit 13 (STEP 104). If the post-meal button 15b is selected, the control unit 14 calculates calories absorbed after a meal from an intensity of light received by the light receiving unit 14 and stores the calorie information in the post-meal data area of the storage unit 13 (STEP 105). The control unit 14 calculates temporal changes in net absorbed calories from the calories absorbed in a fasting period and stored in the reference data area of the storage unit 13 and the calories absorbed after a meal and stored in the post-meal data area of the storage unit 13 (STEP 106).

The absorbed calorie measuring device according to the embodiment is a device such as a smartphone provided with a camera and a flash and comprises a light source intended to irradiate visible light (light source with respect to which the intensity of emitted light having the wavelengths 400 to 800 nm takes up 80% or greater of the intensity of emitted light in all the wavelength regions) and is a health management device by calculating the amount of calorie absorption and/or the amount of consumption from the light attenuation of an irradiation site or the reaching range of constant intensity.

Note that changes till the amount of absorbed calories reaches 0 as shown in Fig. 4 are herein referred to as the amount of absorbed calorie consumption. This calorie consumption rate can be confirmed by changes due to exercise or the like.

The device also enables a user to grasp his/her constitution by inputting the calories of an ingested meal into software and calculating an absorption rate from the amount of absorbed calories. In addition, the device can provide an evaluation of how efficiently lipids were consumed by exercise than during rest, and consequently, it can be used to educate the importance of exercise and adjust the amount of food. It can also be used as an alcohol intake level monitor if the user switches the modes. The user may also derive a reference standard value from the measurement results of a large number of people and change a screen display based on the reference standard value to visually represent his/her physical condition.

Next, an absorbed calorie measuring device 200 according to another embodiment will be described. A smartphone comprising an irradiation unit and a light receiving unit is used and the absorbed calorie measuring device 200 according to the embodiment may be a server device connected to the smartphone via a network.

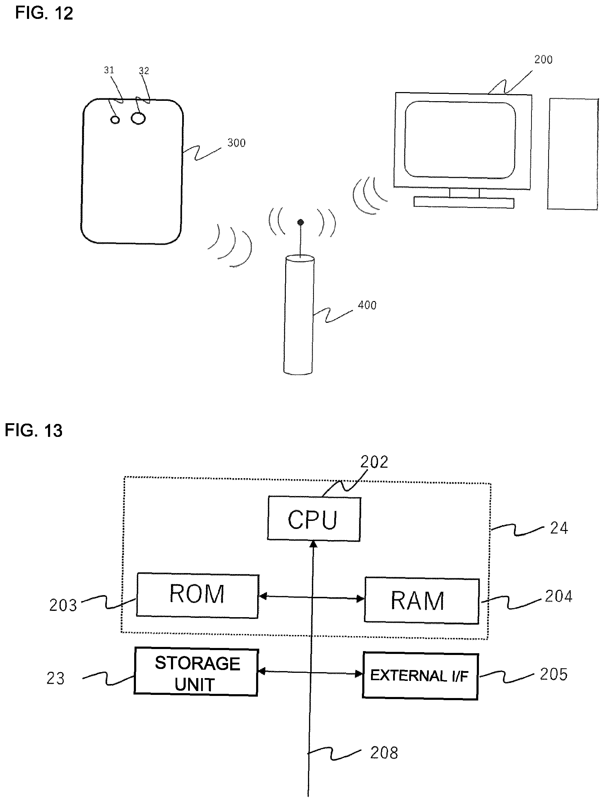

A system configuration diagram according to the embodiment is shown in Fig. 12. The system comprises the absorbed calorie measuring device 200, a mobile terminal 300, and an access point 400.

The mobile terminal 300 comprises an irradiation unit 31, a light receiving unit 32 and an operation panel 35. Since the configuration of the mobile terminal 300 is the same as the hardware configuration of the absorbed calorie measuring device 100 according to the embodiment, the description of its configuration will be omitted.

The absorbed calorie measuring device 200 according to the embodiment is, for example, a server device. The configuration of a control system of the absorbed calorie measuring device 200 according to the embodiment will be described. Fig. 13 is a block diagram of the absorbed calorie measuring device 200 according to the embodiment. A CPU 202, a ROM (Read Only Memory) 203, a RAM (Random Access Memory) 204, an external I/F (Interface) 205, and a storage unit 23 are connected via a system bus 208. The CPU 202, the ROM 203, and the RAM 204 constitute a control unit 24.

The ROM 203 previously stores programs and thresholds to be executed by the CPU 202.

In the RAM 204, various memory areas such as an area for developing a program to be executed by the CPU 202 and a work area for data processing by the program are dynamically formed.

The storage unit 23 is an internal storage such as an SSD (Solid State Drive) or an HDD (Hard Disc Drive).

The storage unit 23 has a reference data area configured to store the information of a subject's calories absorbed in a fasting period, which is a standard value in a fasting period. The control unit 24 calculates absorbed calories based on the information stored in the reference data area.

The storage unit 23 has a post-meal data area configured to store the information of a subject's calories absorbed after a meal over time. The control unit 24 calculates absorbed calories based on the information stored in the post-meal data area.

The storage unit 23 stores correlation data in which a correlation between absorbed calories and the intensity of received light is converted into a calibration curve. In the embodiment, the calories absorbed in a fasting period, the calories absorbed after a meal and the correlation data are stored in the storage unit 23, but they may also be stored in the RAM 204.

The control unit 24 calculates the calories absorbed in a fasting period from an intensity of received light in a fasting period. The control unit 14 calculates the calories absorbed after a meal from an intensity of received light after a meal.

The control unit 24 calculates net absorbed calories from the difference between the calories absorbed after a meal and the calories absorbed in a fasting period.

In the embodiment, the calories absorbed in a fasting period and the calories absorbed after a meal are calculated individually, but obtaining calories is not limited to this calculation. The absorbed calories at 0 minutes of the calories absorbed after a meal in Fig. 4 may be deemed to be the calories absorbed in a fasting period (i.e., a standard value) and the above process may be performed.

The external I/F 205 is an interface for communicating with an external device. It is sufficient if the external I/F 205 is an interface that performs data communication with an external device. For example, the external I/F 205 may be a device (a USB memory or the like) that is locally connected to an external device, or may be a network interface for communicating via a network. Furthermore, its data communication method may be Wi-Fi (registered trademark) communication or USB communication.

The absorbed calorie measuring device 200 having the above configuration executes the absorbed calorie measuring process based on a preset program.

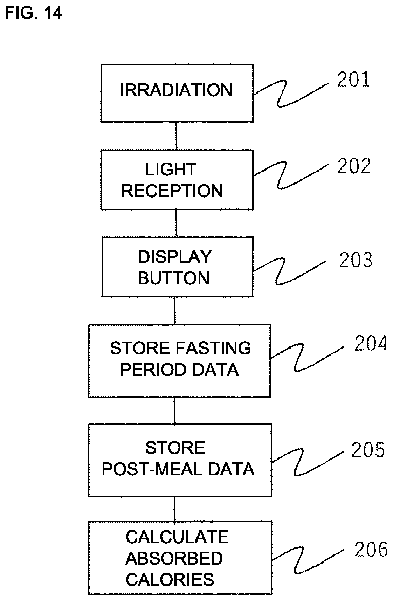

Fig. 14 is a flowchart showing the operation of the absorbed calorie measuring device 200 according to the embodiment. The irradiation unit 31 of the mobile terminal 300 irradiates a subject with irradiation light (STEP 201). The light receiving unit 32 of the mobile terminal 300 receives the light emitted from inside to outside the subject (STEP 202). The operation panel 35 of the mobile terminal 300 displays a fasting period button and a post-meal button (STEP 203). If the fasting period button is selected, the control unit 24 of the absorbed calorie measuring device 200 calculates calories absorbed in a fasting period from an intensity of light received by the light receiving unit of the mobile terminal 300 and stores the calorie information in the reference data area of the storage unit 23 (STEP 204).

If the post-meal button is selected, the control unit 24 of the absorbed calorie measuring device 200 calculates calories absorbed after a meal from an intensity of light received by the light receiving unit of the mobile terminal 300 and stores the calorie information in the post-meal data area of the storage unit 23 (STEP 205). The control unit 24 of the absorbed calorie measuring device 200 calculates temporal changes in net absorbed calories from the calories absorbed in a fasting period and stored in the reference data area of the storage unit 23 and the calories absorbed after a meal and stored in the post-meal data area of the storage unit 23 (STEP 206) .

The embodiments have been described above, but they are presented by way of example and are not intended to limit the scope of the invention. These novel embodiments can be implemented in various other modes, and various omissions, replacements and modifications can be made without departing from the gist of the invention. This embodiment and its modifications are included in the scope and gist of the invention and are also included in the invention described in the claims and the equivalent scope thereof.

Reference Signs List

| | |
|---|---|
| 100 | Absorbed calorie measuring device |
| 11 | Irradiation unit |
| 12 | Light receiving unit |
| 13 | Storage unit |
| 14 | Control unit |
| 15 | Operation panel unit |

**Claims**

1.  An absorbed calorie measuring device comprising:

    an irradiation unit configured to irradiate a subject with light;
    a light receiving unit configured to detect an intensity of received light which passes through the subject and is emitted outside the subject; and
    a control unit configured to calculate absorbed calories from the intensity of received light.

2.  The absorbed calorie measuring device according to claim 1,

    wherein the control unit calculates calories absorbed in a fasting period from an intensity of light received in a fasting period,
    calories absorbed after a meal from an intensity of light received after a meal and
    net absorbed calories from the calories absorbed in a fasting period and the calories absorbed after a meal.

3.  The absorbed calorie measuring device according to claim 2, further comprising:

    an operation panel configured to display a fasting period button and a post-meal button; and
    a storage unit comprising a fasting period data area and a post-meal data area,
    wherein if the fasting period button is selected, the control unit stores the calories absorbed in a fasting period in the fasting period data area and
if the post-meal button is selected, the control unit stores the calories absorbed after a meal in the post-meal data area.

4. The absorbed calorie measuring device according to claim 2 or 3,
wherein the control unit calculates net absorbed calories from the difference between the calories absorbed in a fasting period and the calories absorbed after a meal.

5. The absorbed calorie measuring device according to claim 3,

wherein the storage unit stores statistical data of previously obtained intensities of received light and absorbed calories and
the control unit calculates the calories absorbed in a fasting period and the calories absorbed after a meal from the intensities of received light based on the statistical data stored in the storage unit.

6. The absorbed calorie measuring device according to any one of claims 1 to 5,
wherein the light irradiated by the irradiation unit is visible light.

7. An absorbed calorie measuring program for causing a computer of an absorbed calorie measuring device to execute a process of calculating absorbed calories from an intensity of received light,
the device comprising an irradiation unit configured to irradiate a subject with light and a light receiving unit configured to detect the intensity of received light which passes through the subject and is emitted outside the subject.

8. An absorbed calorie measuring method for causing a computer of an absorbed calorie measuring device to execute a process of calculating absorbed calories from an intensity of received light,
the device comprising an irradiation unit configured to irradiate a subject with light and a light receiving unit configured to detect the intensity of received light which passes through the subject and is emitted outside the subject.

9. An absorbed calorie measuring device connected to a mobile terminal, the device comprising a control unit configured to calculate absorbed calories from an intensity of received light,
the mobile terminal comprising an irradiation unit configured to irradiate a subject with light and a light receiving unit configured to detect the intensity of received light which passes through the subject and is emitted outside the subject.

10. The absorbed calorie measuring device according to claim 9,

wherein the control unit calculates calories absorbed in a fasting period from an intensity of light received in a fasting period,
calories absorbed after a meal from an intensity of light received after a meal and
net absorbed calories from the calories absorbed in a fasting period and the calories absorbed after a meal.

11. The absorbed calorie measuring device according to claim 10,

wherein the mobile terminal further comprises an operation panel configured to display a fasting period button and a post-meal button and
the absorbed calorie measuring device further comprises a storage unit comprising a fasting period data area and a post-meal data area,
wherein if the fasting period button is selected, the control unit stores the calories absorbed in a fasting period in the fasting period data area and
if the post-meal button is selected, the control unit stores the calories absorbed after a meal in the post-meal data area.

12. The absorbed calorie measuring device according to claim 10 or 11,
wherein the control unit calculates net absorbed calories from the difference between the calories absorbed in a fasting period and the calories absorbed after a meal.

13. The absorbed calorie measuring device according to claim 11,

wherein the storage unit stores statistical data of previously obtained intensities of received light and absorbed calories and

the control unit calculates the calories absorbed in a fasting period and the calories absorbed after a meal from the intensities of received light based on the statistical data stored in the storage unit.

14. The absorbed calorie measuring device according to claims 9 to 13,
    wherein the light irradiated by the irradiation unit is visible light.

15. An absorbed calorie measuring program for causing a computer of an absorbed calorie measuring device connected to a mobile terminal to execute a process of calculating absorbed calories from an intensity of received light,
    the mobile terminal comprising an irradiation unit configured to irradiate a subject with light and a light receiving unit configured to detect the intensity of received light which passes through the subject and is emitted outside the subject.

16. An absorbed calorie measuring method for causing a computer of an absorbed calorie measuring device connected to a mobile terminal to execute a process of calculating absorbed calories from an intensity of received light,
    the mobile terminal comprising an irradiation unit configured to irradiate a subject with light and a light receiving unit configured to detect the intensity of received light which passes through the subject and is emitted outside the subject.

FIG. 1

FIG. 2

FIG. 3

FIG. 4

FIG. 5

SEX ● MALE ○ FEMALE

AGE [ 50 ] YEARS OLD

HEIGHT [ 170 ] c m

WEIGHT [ 65 ] k g

AMOUNT OF FOOD [ 1226 ] kcal

MEASUREMENT MODE

FIG. 6

MAXIMUM ABSORBED CALORIES

806 kcal

ABSORPTION RATE

65.7 %

CURRENT ABSORBED CALORIES

326 kcal

CURRENT CONSUMPTION RATE

59.6 %

COMMENTS

FIG. 7

FIG. 8

FIG. 9

FIG. 10

FIG. 11

FIG. 12

FIG. 13

FIG. 14

```
┌─────────────────┐
│   IRRADIATION   │ ⟍ 201
└─────────────────┘
         │
┌─────────────────┐
│      LIGHT      │ ⟍ 202
│    RECEPTION    │
└─────────────────┘
         │
┌─────────────────┐
│     DISPLAY     │ ⟍ 203
│     BUTTON      │
└─────────────────┘
         │
┌─────────────────┐
│  STORE FASTING  │ ⟍ 204
│   PERIOD DATA   │
└─────────────────┘
         │
┌─────────────────┐
│      STORE      │ ⟍ 205
│  POST-MEAL DATA │
└─────────────────┘
         │
┌─────────────────┐
│    CALCULATE    │ ⟍ 206
│    ABSORBED     │
│    CALORIES     │
└─────────────────┘
```

**INTERNATIONAL SEARCH REPORT**

| International application No. |
|---|
| PCT/JP2019/050982 |

| A. CLASSIFICATION OF SUBJECT MATTER |
|---|
| Int. Cl. G01N21/49(2006.01)i, G16H20/60(2018.01)i<br>FI: G01N21/49 A, G16H20/60 |
| According to International Patent Classification (IPC) or to both national classification and IPC |

| B. FIELDS SEARCHED |
|---|
| Minimum documentation searched (classification system followed by classification symbols)<br>Int. Cl. G01N21/00-21/01, G01N21/17-21/61, A61B5/145-5/1464, G06Q50/22,<br>G16H20/60 |
| Documentation searched other than minimum documentation to the extent that such documents are included in the fields searched<br>Published examined utility model applications of Japan    1922-1996<br>Published unexamined utility model applications of Japan  1971-2020<br>Registered utility model specifications of Japan         1996-2020<br>Published registered utility model applications of Japan  1994-2020 |
| Electronic data base consulted during the international search (name of data base and, where practicable, search terms used) |

| C. DOCUMENTS CONSIDERED TO BE RELEVANT |  |  |
|---|---|---|
| Category* | Citation of document, with indication, where appropriate, of the relevant passages | Relevant to claim No. |
| X<br><br>Y | US 2017/0360303 A1 (SAMSUNG ELECTRONICS CO., LTD.)<br>21 December 2017, paragraphs [0050]-[0100], fig.<br>1-6, paragraphs [0050]-[0100], fig. 1-6 | 1-2, 4, 7-10,<br>12, 15-16<br>3, 5-6, 11,<br>13-14 |
| Y | JP 2017-523832 A (SANOFI-AVENTIS DEUTSCHLAND GMBH)<br>24 August 2017, paragraphs [0030], [0041]-[0062],<br>fig. 1-4 | 3, 5-6, 11,<br>13-14 |
| Y | JP 2002-510515 A (THE GENERAL HOSPITAL<br>CORPORATION) 09 April 2002, paragraph [0048] | 6, 14 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05.03.2020 | 17.03.2020 |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

**INTERNATIONAL SEARCH REPORT**

International application No.

PCT/JP2019/050982

C (Continuation).   DOCUMENTS CONSIDERED TO BE RELEVANT

| Category* | Citation of document, with indication, where appropriate, of the relevant passages | Relevant to claim No. |
|---|---|---|
| Y | CN 108670269 A (BEIJING INSTITUTE OF TECH SHENZHEN RESEARCH INSTITUTE) 19 October 2018, paragraphs [0005]-[0013], [0018]-[0032], fig. 1-3 | 6, 14 |
| A | WO 2018/151150 A1 (MEDICAL PHOTONICS CO., LTD.) 23 August 2018, entire text, all drawings | 1-16 |
| A | JP 2003-50200 A (NIKKISO CO., LTD.) 21 February 2003, entire text, all drawings | 1-16 |
| A | US 2003/0144582 A1 (COHEN, Carl) 31 July 2003, entire text, all drawings | 1-16 |

Form PCT/ISA/210 (continuation of second sheet) (January 2015)

International application No.

PCT/JP2019/050982

| Patent Documents referred to in the Report | Publication Date | Patent Family | Publication Date |
|---|---|---|---|
| US 2017/0360303 A1 | 21.12.2017 | CN 107518875 A paragraph [0051]-[0106], fig. 1-6 KR 10-2017-0142037 A | |
| JP 2017-523832 A | 24.08.2017 | US 2017/0213009 A1 paragraphs [0043], [0073]-[0094], fig. 1-4 CN 106687964 A | |
| JP 2002-510515 A | 09.04.2002 | US 2002/0082487 A1 paragraph [0064] | |
| CN 108670269 A | 19.10.2018 | (Family: none) | |
| WO 2018/151150 A1 | 23.08.2018 | (Family: none) | |
| JP 2003-50200 A | 21.02.2003 | (Family: none) | |
| US 2003/0144582 A1 | 31.07.2003 | WO 2003/023356 A2 entire text, all drawings | |

Form PCT/ISA/210 (patent family annex) (January 2015)

**REFERENCES CITED IN THE DESCRIPTION**

**Patent documents cited in the description**

- JP 2016081407 A
- JP 2012203834 A